(19) Europäisches Patentamt
European Patent Office
Office européen des brevets

(11) **EP 2 706 112 A1**

(12) **EUROPEAN PATENT APPLICATION**

(43) Date of publication:
**12.03.2014 Bulletin 2014/11**

(51) Int Cl.:
***C12M 3/04*** *(2006.01)*

(21) Application number: **12183279.4**

(22) Date of filing: **06.09.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Université Catholique de Louvain 1348 Louvain-La-Neuve (BE)**

(72) Inventors:
• **Vanzieleghem, Thomas**
  **1360 Perwez (BE)**
• **Mahillon, Jacques**
  **1457 Nil-Saint-Vincent (BE)**

• **Jeanmart, Hervé**
  **5100 Jambes (BE)**
• **Degand, Simon**
  **1030 Bruxelles (BE)**
• **Dupont, Christine**
  **1340 Ottignies (BE)**
• **Ladeuze, Sandy**
  **5170 Lesve (BE)**

(74) Representative: **De Clercq, Ann G. Y. et al De Clercq & Partners Edgard Gevaertdreef 10a 9830 Sint-Martens-Latem (BE)**

(54) **Fluidic device for studying of surface-dwelling multicellular layers and microbial biofilms**

(57) The present invention relates to a fluidic device that can be used for the analysis of +surface-dwelling multicellular layers, some of which comprise biofilm and can be referred to as biofilms, and their formation under controlled dynamic conditions. More particularly, the surface in the fluidic chamber on which the multicellular layer is grown is detachable and/or removable from the fluidic chamber, thereby providing a highly versatile device.

Figure 1

## Description

### FIELD OF THE INVENTION

The present invention relates to a fluidic device that can be used for the analysis of surface-dwelling multicellular layers and their formation under controlled dynamic conditions. More particularly, the surface in the fluidic chamber on which the multicellular layer is grown is detachable and/or removable from the fluidic chamber, thereby providing a highly versatile device.

### BACKGROUND

Surface dwelling multicellular layers such as for instance Biofilms are complex communities enmeshed in a self-produced matrix. In nature, biofilms represent about 90% of the bacterial lifestyle since it offers protection against all types of environmental threats (e.g. antibiotics, sanitizing agents). Biofilms are also intimately bound to human activities with both desired (wastewater treatment and gasification) and undesired aspects (corrosion, food intoxication or chronic infections). Biofilm research has gained increasing attention over the past years and this trend will certainly continue in the following years.

For observing and monitoring cellular biological processes (e.g. biofilms), cell-based assays are required where the behavior of living cells and changes in cell behavior in response to various conditions and agents may be observed, for instance through imaging techniques (visualization of microscopic processes through the use of agents that provide detectable signals representative of cellular components or events). Typically, these observations are performed in microtiter plates that contain the cells that can be observed through a microscope. However, these stationary conditions only offer a very narrow range of settings to observe biofilms and hence they do not provide a realistic image when it comes to monitoring biofilms because in nature these systems are usually formed in more dynamic environments, which require simulation of the actual real-life situations.

Formation and monitoring of a multicellular layer is a complex process where many factors (e.g. microorganisms, fluid flow and type, surface material and structure) play a role. Therefore, it is critical to perform the growth of multicellular layers in a versatile device that allows all these factors to be included into the simulation.

In view of the foregoing, there is a need for devices to perform assays, such as biochemical and/or cellular assays that provide for flow of fluid throughout the device so that nominally adhering substances, such as the cells in the multicellular layers can be monitored in *in vitro* conditions close to actual real-life situations.

While several tools enabling the analysis and study of multicellular layers are available, there seems to be a need for tools that act in a more accurate and more versatile manner.

## SUMMARY OF THE INVENTION

The present invention relates to a fluidic device for cultivation and/or analysis of surface dwelling multicellular layers comprising:

- a fluidic chamber comprising one or more surfaces enclosing the fluidic chamber through which fluid can flow along a flow path, wherein said fluidic chamber is characterized by having a Young's Modulus [E] ranging between 500 kPa and 1 MPa;
- an inlet port fluidically connected to said fluidic chamber;
- an outlet port fluidically connected to said fluidic chamber;

characterized therein that said fluidic chamber comprises at least one detachable and/or removable surface for the cultivation and/or analysis of surface dwelling multicellular layers. Surface-dwelling multicellular layers as referred to herein relate to biological monolayer or multilayer structures comprising one or more types of mammalian cells, insect cells, yeast cells, fungal cells, plant cells, microbial cells, bacterial cells, cellular vesicles, cellular organelles or tissue sections. In particular embodiments said multicellular layer composed of microbial cells is a biofilm.

More particularly, the flow of the fluid through said fluidic chamber is laminar, preferably having a Reynolds number smaller than 100. More particularly, the flow of the fluid through said fluidic chamber provides a linear flow profile over the entire width of the fluidic chamber, with the exception of the area (10%) adjacent to the side walls.

In a particular embodiment, the fluidic device according to the present invention provides in a device wherein said fluidic chamber comprises a bottom surface, two side surfaces and a top surface, wherein at least part of said top or bottom surface are detachable and/or removable. More particularly, at least part of said fluidic chamber is transparent for optical imaging, for microscopy, and/or for fluorescence imaging, thereby providing an imaging observation site. More particularly, the fluidic device further comprising observation zones and/or sensors located on or in one or more surface enclosing said fluidic chamber, for observing and/or monitoring the formation and/or cultivation of surface-dwelling multicellular layers on said detachable and/or removable surface. More particularly, said detachable and/or removable surface is made from or provided with an adherent surface material suitable for the adherence of the multicellular layer, wherein said surface material models a surface likely to be involved in cell adhesion and/or multicellular layer formation.

In a particular embodiment, the fluidic device according to the present invention provides that said surface-dwelling multicellular layer adherent surface material is chosen from the group comprising aluminum, stainless steel, silver, copper, hydroaxyapatite, silicon, latex, urethane, PVC, ceramic, steel, gold, titanium, polyethylene, polysiloxanes, biocompatible glasses, poly-methylmethacrylate, Teflon (or PTFE), polypropylene, polystyrene, polyamides, polyethers, polyesters, coated block polymers of polyethylene oxide (PEO), polypropylene oxide (PPO), polybutylene oxide (PBO), food film polymers polycarbonate filters and minerals.

In a further particular embodiment, the fluidic device according to the present invention provides that said fluidic device further comprising an inlet flow distributor for distributing incoming fluid over the fluidic chamber and optionally an outlet flow distributor for guiding the outflowing fluid from the fluidic chamber to the outlet. More particularly, said inlet flow distributor or part thereof slopes downward relative to the horizontal position of the fluidic chamber.

In a further particular embodiment, the fluidic device according to the present invention provides that the depth of the fluidic chamber ranges between 0.1 and 5 mm. More particularly, said fluidic device is made from a polymeric material and wherein said detachable and/or removable surface wall of said fluidic device is made from a material other than said polymeric material.

In a further embodiment, the present invention relates to a method for cultivating and monitoring surface-dwelling multicellular layers, comprising:

a) providing a fluidic device comprising:

- a fluidic chamber comprising one or more surfaces enclosing the fluidic chamber through which fluid can flow along a flow path, said fluidic chamber comprising at least one detachable and/or removable surface for cultivating multicellular layers;
- an inlet port fluidically connected to said fluidic chamber;
- an outlet port fluidically connected to said fluidic chamber;

b) dispensing a flowing liquid growth medium, optionally comprising microorganisms, animal cells, plant cells or fungi cells, into said fluidic chamber, said growth medium flowing across said detachable and/or removable surface, thereby generating a surface-dwelling multicellular layer on said detachable and/or removable surface; and

c) monitoring the multicellular layer on said detachable and/or removable surface under varying conditions.

In particular embodiments said surface dwelling multicellular layer composed of unicellular organism cells (e.g. microbes) is a biofilm.

More particularly the method provides that said varying conditions comprise different types of fluid media, different fluid flow rates, different temperatures and/or combinations thereof.

In a further embodiment, the present invention relates to a flow distributor for distributing a fluidic flow from a narrow inlet channel to a wider fluidic channel, wherein said flow distributor comprises a first distribution region shaped as an isosceles trapezoid fluidically connected through the larger of the two parallel sides of said isosceles trapezoid to a second rectangular shaped distribution region, wherein the narrow inlet channel is fluidically connected to the smaller of the two parallel sides of said first distribution region and said wider fluidic channel is fluidically connected to the second distribution region, **characterized in that** said second distribution region slopes downward relative to the horizontal position of said wider fluidic channel.

## BRIEF DESCRIPTION OF THE DRAWING

The following description of a specific embodiment of the invention is merely exemplary in nature and is not intended to limit the present teachings, their application or uses.

**Figure 1** Figure 1 illustrates a specific embodiment of the fluidic device according to the present invention.
**Figure 2** Figure 2 illustrates fluidic simulations (Fluent) in a fluidic device according to an embodiment of the present invention
**Figure 3** Figure 3 illustrates a specific embodiment of the fluidic device according to the present invention.

## DETAILED DESCRIPTION

The present invention will be described with respect to particular embodiments but the invention is not limited thereto.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or openended and do not exclude additional, non-recited members, elements or method steps. The terms "comprising", "comprises" and "comprised of" when referring to recited members, elements or method steps also include embodiments, which "consist of" said recited members, elements or method steps.

Furthermore, the terms first, second, third and the like in the description, are used for distinguishing between similar elements and not necessarily for describing a sequential or chronological order, unless specified. It is to be understood that the terms so used are interchangeable under appropriate circumstances and that the embodiments described herein are capable of operation in other sequences than described or illustrated herein.

All documents cited in the present specification are hereby incorporated by reference in their entirety.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art.

The present invention provides in a fluidic device providing in the growth of a surface-dwelling multicellular layer under controlled dynamic conditions. The device allows the cultivation and growth of a multicellular layer on a surface adherent material, but also offers the possibility to monitor and analyze the multicellular layer under changing and controlled conditions. The contact interface on which the multicellular layer is grown is removable and/or detachable from the fluidic chamber, thereby being adaptable and allowing alteration. Also the control of the fluid flow is highly regulated throughout the fluid cell and the device offers a high flexibility in analysis possibilities. In addition, all system parts of the fluidic device are autoclavable, a property of major importance with regard to biosafety and environmental issues. In particular embodiments said multicellular layer is a biofilm.

Surface-dwelling multicellular layers as referred to herein relate to biological monolayer or multilayer structures comprising one or more types of mammalian cells, insect cells, yeast cells, fungal cells, plant cells microbial cells, bacterial cells, cellular vesicles, cellular organelles or tissue sections. Typically, the cultivation and/or growth of surface-dwelling multicellular layers is typically known for the growth of bacterial, archaeal or yeast cells in the form of biofilms. However, also other cell type growth and adhesion may be assessed such as for instance in a particular embodiment the adhesion of animal cells upon a layer of a chosen compound (i.e.

antibodies, receptors, extracellular matrix molecules, biomaterial...). Also, in another particular embodiment fungi and/or plant cells could also be used in the devices according to the present invention.

Accordingly, useful cells include prokaryotes and eukaryotes such as mammalian cells including hybridoma cells, insect cells, yeast cells, fungal cells, plant cells, microbial cells, bacterial cells, tissue sections and/or protist cells comprising protozoa, algae and fungal cells. Mammalian cells may be derived from any recognized source with respect to species (e.g. human, rodent, simian), tissue source (brain, liver, lung, heart, kidney, skin, muscle) and cell type (e.g. epithelial, endothelial). In addition, cells which have been transfected with recombinant genes may also be cultured using the present invention.

Suitable cell lines may be comprised within e.g. the American Type Culture Collection and the German Collection of Microorganisms and Cell Cultures.

Non-limiting examples of useful mammalian cell lines include animal and human cell lines such as Chinese hamster ovary (CHO) cells, Chinese hamster lung (CHL) cells, baby hamster kidney (BHK) cells, COS cells, HeLa cells, THP cell lines, Jurkat cells, hybridoma cells, carcinoma cell lines, hepatocytes, primary fibroblasts, endothelial cells, tumor cell lines and the like. In particular embodiments tumor cell lines or carcinoma cell lines are envisaged.

Suitable insect cell lines include but are not limited to Lepidoptera cell lines such as *Spodoptera frugiperda* cells and *Trichoplusia ni* cells.

Non-limiting examples of fungal cells useful in the present invention include the phyla *Ascomycota, Basidiomycota, Chytridiomycota,* and *Zygomycota* as well as the *Oomycota* and all mitosporic fungi. Representative groups of *Ascomycota* include, e.g., *Neurospora* spp., *Eupenicillium* (or *Penicillium*) spp., *Emericella* (or *Aspergillus*) spp., *Eurotium* (or *Aspergillus*) spp., and the true yeasts listed above. Examples of *Basidiomycota* include mushrooms, rusts, and smuts. Representative groups of *Chytridiomycota* include, e.g., *Allomyces* spp., *Blastocladiella* spp., *Coelomomyces* spp., and aquatic fungi. Representative groups of *Oomycota* include, e.g. saprolegniomycetous aquatic fungi (water molds) such as *Achlya* spp.. Examples of mitosporic fungi include *Aspergillus* spp., *Penicillium* spp., *Candida* spp. and *Altemaria* spp.. Representative groups of *Zygomycota* include, e.g., *Rhizopus* spp. and *Mucor* spp..

Fungal cells may be yeast cells. Non-limiting examples of useful yeast cells include ascosporogenous yeast (*Endomycetales*), basidiosporogenous yeast, and yeast belonging to the Fungi Impeffecti or *Deuteromycota* (*Blastomycetes*). The ascosporogenous yeasts are divided into the families *Spermophthoraceae* and *Saccharomycetaceae*. The latter is comprised of four sub-families, *Schizosaccharomycoideae* (e.g., genus *Schizosaccharomyces* including *S. pombe*), *Nadsonioideae, Lipomycoideae,* and *Saccharomycoideae* (e.g., genera *Pichia* including *P. pastoris, P. guillermondii* and *P. methanolio*), *Kluyveromyces* including *K. lactis, K. fragilis* and *Saccharomyces* including *S. carlsbergensis, S. cerevisiae, S. diastaticus, S. douglasii, S. klayveri, S. norbensis* or *S. oviformis*). The basidiosporogenous yeasts include the genera *Leucosporidim, Rhodosporidium, Sporidiobolus, Filobasidium,* and *Filobasidiella.* Yeasts belonging to the Fungi Impeffecti are divided into two families, *Sporobolomycetaceae* (e.g., genera *Sporobolomyces* and *Bullera*) and *Cryptococcaceae* (e.g., genus *Candida* including *C. maltose*). Other useful yeast host cells are *Hansehula polymorpha, Yarrowia lipolytica, Ustilgo maylis.*

Fungal cells may be filamentous fungal cells including all filamentous forms of the subdivision *Eumycota* and *Oomycota.* Filamentous fungi are characterized by a vegetative mycelium composed of chitin, cellulose, glucan, chitosan, mannan, and other complex polysaccharides. Vegetative growth is by hyphal elongation and carbon catabolism is obligatory aerobic. In contrast, vegetative growth by yeasts such as *Saccharomyces cerevisiae* is by budding of a unicellular thallus and carbon catabolism may be fermentative. In a more preferred embodiment, the filamentous fungal host cell is a cell of a species of, but not limited to the genera, *Acremonium, Aspergillus, Fusarium, Humicola, Mucor, MyceliopHthora, Neurospora, Penicillium, Thielavia, Tolypocladium,* and *Trichoderma* or a teleomorph or synonym thereof.

Suitable plant cells for use in the present invention include dicotyledonous plant cells, examples of which are *Arabidopsis thaliana,* tobacco, potato, tomato, and leguminous (e.g. bean, pea, soy, alfalfa) cells. It is, however, contemplated that monocotyledonous plant cells, e.g. monocotyledonous cereal plant cells such as for example rice, rye, barley and wheat, may be equally suitable.

A multicellular layer of adherent microorganisms is often referred to as a biofilm. The microorganisms in the biofilm may include a mixture of bacteria, for example, including Gram-positive, Gram-negative bacteria, anaerobic bacteria, aerobic bacteria, and any combination thereof. The Gram-positive bacteria includes without limitation to *Enterococcus faecalis, Staphylococcus aureus, Staphylococcus epidermidis, Streptococcus viridans, Listeria monocytogenes, Bacillus cereus* or any combination thereof. The Gram-negative bacteria includes without limitation to *Escherichia coli, Klebsiella pneumoniae, Proteus mirabilis, Pseudomonas aeruginosa* and any combination thereof. Bacteria may be aerobic or anaerobic, the anaerobic bacteria is *Streptococcus anginosus, Streptococcus australis, Streptococcus constellatus, Streptococcus mitis, Enterobacter* spp., *Actinomyces* spp., *Veillonella* spp., *Prevotella melaninogenica, Fusobacterium periodonticum* or any combination thereof. The aerobic bacteria includes without limitation to strains fo the following genera *Aeromonas, Bacillus, Burkholderia, Flavobacterium, Listeria, Microbacterium, Pseudomonas, Salmonella, Staphylococcus* or any combination thereof. In certain embodiments, the biofilm includes oral bacteria. The oral bacteria includes without limitation to *Actinomyces viscosus, Actinomyces naselundii, Streptococcus mutans, Streptococcus sanguis, Streptococcus sobrinus, Lactobacillus casei, Lactobacillus acidophilius, Candida albicans, Actinobacillus actinomycetemcomitans, Veillonella parvula, Fusobacterium nucleatum* subsp. *polymorphum, Porphyromonas gingivalis, Neisseria* spp., and any combination thereof.

In particular embodiments said multicellular layers as referred to herein comprise a combination of two or more different types of organisms such as for instance the combination of bacteria with yeast or the combination of human cell lines with bacteria.

The removable and/or detachable contact interface in the fluidic chamber provides that the contact surface can be removed from the fluidic chamber without the necessity to break parts of it or without having to apply forces that could disrupt the fore grown multicellular layer.

The devices and methods according to the present invention accordingly allow the cultivation of surface-dwelling multicellular layers under controlled dynamic flow conditions where these parameters are controlled over the entire scan observation area, thereby assuring a laminar flow parabolic velocity profile and constant shear forces over this area. The contact interface on which the multicellular layer is grown is removable and/or detachable from the fluidic chamber and is pressed against the fluidic chamber without the requirement of any other seals. Accordingly, only the contact interface on which the multicellular layer is grown is contaminated by the culture medium. The removable character of this contact interface also allows fast and easy adaptation depending on the needs and the type of multicellular layer to be analyzed. A surface of hydroxyapatite may for instance be used for experimenting with dental biofilms, a stainless steel plate may be used to simulate behavior in industry applications and silicone can be used to mimic the attachment of bacteria (biofilms) on biomaterials. When using observations zones for monitoring the multicellular layer, the observation may occur in real time depending on the properties of the surface to be used. Also, once the experiments are performed, the biomass of the surface-dwelling multicellular layer can easily be recovered as the surface can be removed and subjected to further surface analysis. Also, the temperature in the growth chamber is kept constant through good heat diffusion. If accurately calibrated, a simple equipment such as a Peltier or a hot plate is sufficient to maintain an optimized temperature control. The medium passing through the flow chamber is heated at the desired temperature.

In a first aspect, the present invention relates to a fluidic device for cultivation and/or analysis of surface-dwelling multicellular layers comprising:

- a fluidic chamber comprising one or more surfaces enclosing the fluidic chamber through which fluid can flow along a flow path;

- an inlet port fluidically connected to said fluidic chamber;
- an outlet port fluidically connected to said fluidic chamber;

characterized therein that said fluidic chamber comprises at least one detachable and/or removable surface for the cultivation and/or analysis of surface-dwelling multicellular layers.

In particular embodiments said multicellular layer composed of microbial cells is a biofilm.

Compared to other flow cell systems for growing multicellular layers such as biofilms in dynamic conditions, the fluidic device according to the present invention is characterized by having at least one interchangeable contact surface for the growth of the multicellular layer. Indeed the surface, on which the multicellular layer is cultivated, is removable and/or detachable from the fluidic device. This allows the user to change the cultivation surface depending on the type of surfaces one wants to study the multicellular layer formation on. Accordingly, the surface-dwelling multicellular layer formation is not limited to the type of material the fluidic chamber is made of. As one of the surfaces is detachable, the material from which this surface is made can be chosen by the user. Also, the detachable and/or removable surface for the cultivation and/or analysis of multicellular layers does not compromise the type of analysis options using for instance microscopy, spectroscopy or the recovery of biomass.

Also, the fluidic device according to the present invention offers the possibility to grow surface-dwelling multicellular layers in dynamic conditions under controlled conditions of the fluidic flux inside the fluidic chamber, thereby obtain the desired accuracy and reproducibility.

Alternatively, the devices according to the present invention may also be used to test the anti-fouling properties of a surface. Here fouling on the detachable and/or removable surface can be assessed. As the formation of fouling on a surface is correlated with biofilm formation on that surface, both uses of the devices according to the present invention are closely related.

As used herein, the term "fluidic chamber" refers to an enclosure that defines an interior space or cavity which can contain a fluid and through which a fluid can flow. The fluid is referred to as a substance that has the ability to flow and to conform to the boundaries of a container in which the substance is placed. Fluids include liquid and gas substances, and preferably liquid substances. Fluids include, but are not limited to, water, buffer, blood, preparative fluids, culture medium, reagents, organic solutions, inorganic solutions, and any fluid that can be used to conduct an assay.

The fluid in the fluidic device according to the present invention flows through the fluidic chamber according to a flow path (the path of flow of a fluid). A flow path can be the path of flow of a fluid through a flow chamber. For example, a flow path can be the path of flow of a fluid from an inlet port, through the interior of a chamber and to an outlet port. As used herein "inlet port" refers to a port through which a substance, e.g., a fluid, is introduced into a chamber, whereas "outlet port" refers to a port through which a substance, e.g., a fluid, is removed from a chamber. As used herein "fluidically connected" indicates that the items referred to are in fluid communication with each other.

In a particular embodiment, the fluidic device according to the present invention provides that the flow of the fluid through said fluidic chamber is laminar. More particularly, the Reynolds number of the flow of the fluid through said fluidic chamber is smaller than 100.

As used herein, the term "laminar" or "laminar flow", with reference to a fluid, refers to the fluid flow being substantially non-turbulent. The degree of laminar flow can be characterized by the Reynolds number, which is a measurement of the tendency for turbulence to occur as described by the equation:

$$Re = \frac{\rho . v . L}{\mu}$$

wherein Re is a Reynolds number, v is the average linear flow rate, L is a unit of length, $\rho$ is the density of the fluid and $\mu$ is dynamic viscosity of the fluid ($\mu/\rho$ being the kinematic viscosity of the fluid). Turbulence in fluid flow occurs in flows characterized by high Reynolds (Re) numbers. Thus, laminar flow generally can be characterized by a Re less than about 100. In particular embodiments, laminar flow can be characterized by a Re less than about 90, or less than about 80, or less than about 70, or less than about 60, or less than about 50, or less than about 40, or less than about 30, or less than about 20 or less than about 10. Laminar flow can also be referred to as a streamlined, uniform flow of fluid near a solid boundary without any significant turbulence. A fluid path that has little to no bending, little to no sharp turns, little to no steep slopes, little to no recirculation paths, little to no retroflow, little to no eddy currents, and little to no whorls generally can provide for substantially laminar flow. The term is also used to describe a linear flow of fluid that has a controllable linear velocity at the surface of a flow chamber provided and that is non-disruptive to an element (e.g., a living cell or an analyte) of a process, e.g., a biological or biochemical process, in the flow chamber.

As used herein, substantially laminar flow inside the fluidic chamber, with reference to a fluid, is a flow of fluid that is at least about 80% laminar, at least about 85% laminar, at least about 90% laminar, at least about 95% laminar, at least about 96% laminar, at least about 97% laminar, at least about 98% laminar, at least about 99% laminar, or at least about 100% laminar. The laminar flow in the fluidic chamber avoids the occurrence of dead zones in the chamber. Accordingly, any non attached cells of the multicellular layer are cleared from the fluidic chamber through the constant laminar flow prohibiting them from interfering with the analysis.

For instance, for the flow of water at 30°C in the fluidic chamber of the device according to the present invention (with for instance a characteristic length of 0.001 m and a fluid velocity of 0.001m/s) the calculated Reynolds Number is about 1.3. Since the speed of the liquid has a plain linear contribution to Re (Reynolds Number). Typical fluid flow velocities in the devices according to the present invention do not exceed 0.01 m/s for multicellular layer growth to occur and the density of medium used for the multicellular layer growth about 1000 kg/m$^3$.

In a particular embodiment, the fluidic device according to the present invention provides that the flow in the fluidic chamber has a Hydraulic Retention Time (HRT = Volume [m$^3$]/Flow [m$^3$/s]) ranging between 5 seconds and 30 minutes, more particularly, between 10 seconds and 15 minutes, more particularly, between 15 seconds and 5 minutes, more particularly, between 20 seconds and 60 seconds and more particularly, about 30 seconds. Typical fluid flow rates through the fluidic device range between 0.05 ml/min and 10 ml/min.

In a particular embodiment, the fluidic device according to the present invention provides that the flow of the fluid through said fluidic chamber provides a linear flow profile over the entire width of the fluidic chamber, with the exception of the area 10% from the side walls. More particularly, the present invention provides that the flow of the fluid through said fluidic chamber provides a linear flow profile over the entire width of the fluidic chamber, with the exception of the area at a distance from the side walls which is smaller than 10%, 9%, 8%, 7.5%, 7%, 6.5%, 6.25% or 6% of the total width of the fluidic chamber. Accordingly, the present invention provides that the flow of the fluid through said fluidic chamber provides a linear flow profile over at least 80% of the total width of the fluidic chamber, more particularly, a linear or laminar flow profile is provided over at least 80%, 82%, 84%, 85%, 86%, 87%, 87.5% or 88% of the total width of the fluidic chamber. Accordingly, the fluid passes through the fluidic chamber in a laminar manner thereby generating a fluid flow which provides an equal velocity over the entire width of the fluidic chamber, the only deviation from this occurring in the area near the side walls. Typically the area where the deviation occurs is located in the area from the side walls which is smaller than 10%, 9%, 8%, 7.5%, 7%, 6.5%, 6.25% or 6% of the total width of the fluidic chamber. For instance, when the chamber is 15 mm wide, the area where the deviation occurs is in the 1.5 mm area or less from the side walls.

In a particular embodiment, the fluidic device according to the present invention provides that said fluidic chamber comprises a bottom surface, two side surfaces and a top surface, wherein at least part of said top or bottom surface are detachable and/or removable. More particularly, the detachable and/or removable surface is the bottom surface.

In a particular embodiment, the fluidic device according to the present invention provides that at least part of said fluidic chamber is transparent for optical imaging, for microscopy, and/or for fluorescence imaging, thereby providing an imaging observation site. For instance at least part of the fluidic chamber is made transparent for observation and/or monitoring of the surface-dwelling multicellular layer through classical light microscopy. The fluidic chamber typically provides a sufficient optical transparency and clarity to permit observation of the multicellular layer and the cells thereof. The transparent characteristic of part of the fluidic chamber is especially useful for real time imaging of the multicellular layer.

In a particular embodiment the imaging of the surface-dwelling multicellular layer occurs using a confocal microscope thereby permitting 3D images to be made.

More particularly, at least part of the fluidic chamber is casted in a transparent or translucid, heat-resistant, polymeric material which Young's Modulus [E] is comprised between 500 kPa and 1 MPa. The rigidity is an important parameter for the device according to the present invention as the removable surface of the fluidic chamber is pressed against the rest of the device to ensure complete sealing of the fluidic chamber. This makes materials with a too low Young's modulus unsuitable because the open flow chamber inside the material would lose its shape under pressure applied to seal the device. Accordingly, materials such as PDMS (Poly-dimethylsiloxane) (good rigidity, transparency and heat-resistance up to 300°C) are used.

The plates ensuring the pressure that maintains the removable surface and the fluidic chamber together can be made of any hard (Young modulus superior to 10 GPa), heat resistant material such as steel, aluminum or plastic polymer. The pressure can be provided through any commonly known method in the art.

In a particular embodiment, the fluidic device according to the present invention further comprises observation zones and/or sensors located on or in one or more surface enclosing said fluidic chamber, for observing and/or monitoring the formation and/or cultivation of the surface-dwelling multicellular layer on said detachable and/or removable surface.

As referred herein the "observation zones" and/or "sensors" are defined features in the walls of the fluidic chamber that allow observation using commonly known detection methods, such as for instance observation through (real-time) light microscopy. More particularly, one of the walls of the fluidic chamber is provided with an array of "observation fields" displaying the same set of conditions. Pictures taken from various observation fields can be statistically treated as repetitions of the same experiment. Observation fields positioned along different flow path lines can be considered independent repetitions of the same experiment.

In a particular embodiment of the present invention the sensors are crafted on the surface, said sensors being chosen from, but not limited to, for example, microelectrodes measuring gas concentration, pH, nutrient levels and redox potential of the local environment.

Another advantage of the removable and/or detachable surface is that upon finalization of the experiment, the surface on which the multicellular layer has grown may be detached and recovered, preferably sterilely, for further analysis. Accordingly, of the detached surface properties such as surface composition, biomass recovery, and mechanical properties assessment (rheometry) can be assessed using adequate methods and techniques such as XPS or mass spectrometry (for analysis of the surface composition), Rheometer electron microscopy (for assessment the mechanical properties). The detached surface may also be treated in various ways to assess the resistance of multicellular layers towards a certain set of conditions, an aspect which is useful when the treatment conditions are unsuitable for passing through the fluidic device.

In a particular embodiment, the fluidic device according to the present invention provides that said detachable and/or removable surface is made from or provided with a surface-dwelling multicellular layer adherent surface material, wherein said surface material models a surface likely to be involved in cell adhesion and/or formation of a surface-dwelling multicellular layer. As multicellular layer formation on a surface is closely linked to the formation of fouling, the devices according to the present invention also envisage the analysis of the anti-fouling properties of a surface. Therefore fouling on the detachable and/or removable surface can be assessed by using a surface material likely to reject and withhold cell adhesion and/or multicellular layer formation (e.g Pluronics coatings (brush polymers)).

In a particular embodiment, the fluidic device according to the present invention provides that said adherent surface material is chosen from the group comprising aluminum, stainless steel, silver, copper, hydroaxyapatite, silicon, latex, urethane, PVC, and ceramic, steel, gold, titanium, polyethylene, polysiloxanes, biocompatible glasses, poly-methylmethacrylate (PMMA-contact lenses material), Teflon (or PTFE), polypropylene, polystyrene, polyamides, polyethers, polyesters, coated block polymers of polyethylene oxide (PEO), polypropylene oxide (PPO), polybutylene oxide (PBO) known to be pluronics used as anti-adhesive coatings, hydrogels, food film polymers, polycarbonate filters (to study the effect of the diffusion of a molecule on the multicellular layer coming from an underlying solution or agar medium) or minerals (to study biofilm formation on river rocks).

Alternatively, the fluidic device according to the present invention provides that said detachable and/or removable surface is provided with an adherent surface material comprising proteins, glycoproteins (e.g. mucins) antibodies, DNA, polysaccharides, lipids or other biomolecules for the purpose of adhering cells and/or other components.

The type of surface material is typically dependant on the type of sterilization that is required. For sterilization through autoclaving, envisaged surfaces are surfaces resistant to humid heat (121°C for at least 20 minutes) such as for instance glass surfaces topped with all sorts of coatings such as for instance metal deposited by CVD (chemical vapor deposition), polymers layered thereupon, sprayed ions, mineral layers (hydroxyapatite) or other bulk materials such as steel, alloys, metals, mineral rocky materials and polymers so long as their mechanical rigidity is sufficient (Young modulus [E] above 10 GPa).

For other sterilization methods (e.g. Gamma rays, electron beams, UV, ethylene oxide, ...) only the mechanical rigidity (E>10GPa) forms a structural limit. Alternatively, the surface material is backed up by a more resistant supporting material providing the structure with the required mechanical rigidity.

In a particular embodiment, the fluidic device according to the present invention further comprises an inlet flow distributor for distributing incoming fluid over the fluidic chamber and optionally an outlet flow distributor for guiding the outflowing fluid from the fluidic chamber to the outlet. More particularly, the flow distributor distributes the incoming fluid flow evenly over the fluidic chamber.

More particularly, the inlet flow distributor or part thereof slopes downward relative to the horizontal position of the fluidic chamber. More particularly, the slope angle relative to the direction of the inlet port or the fluidic chamber ranges between 15° and 75°, more particularly between 30° and 60°, more particularly between 40° and 50° and more particularly, about 45°.

In a particular embodiment the slope angle relative to the direction of the inlet port or the fluidic chamber of the bottom surface is larger compared to the slope angle relative to the direction of the inlet port or the fluidic chamber of the top surface. More particularly, the slope angle relative to the direction of the inlet port or the fluidic chamber of the top surface ranges between 15° and 75°, more particularly between 30° and 60°, more particularly between 40° and 50° and more particularly, about 45°, whereas the slope angle relative to the direction of the inlet port or the fluidic chamber of the bottom surface is larger than 50°, 60°, 75°, 80°, 85° and more particularly, about 90°.

In a particular embodiment the slope angle relative to the direction of the inlet port or the fluidic chamber of the top surface is 45 °, while the slope angle relative to the direction of the inlet port or the fluidic chamber of the bottom surface is 90°.

The angles of the top and bottom surfaces of the flow distributor are chosen to allow an even and efficiently spread of the liquid in the fluidic chamber, while maintaining a good hydrodynamic profile without the need to lengthen the device.

In a particular embodiment, the fluidic device according to the present invention provides that the depth of the fluidic chamber ranges between 0.1 and 5 mm.

In a particular embodiment, the fluidic device according to the present invention provides that said fluidic device is made from a polymeric material and wherein said detachable and/or removable surface wall of said fluidic device is made from a material other than said polymeric material. More particularly, said polymeric material is PDMS.

The present invention further provides in a method for cultivating and monitoring surface-dwelling multicellular layers comprising:

a) providing a fluidic device comprising:

- a fluidic chamber comprising one or more surfaces enclosing the fluidic chamber through which fluid can flow along a flow path, said fluidic chamber comprising at least one detachable and/or removable surface for the cultivating surface-dwelling multicellular layers;
- an inlet port fluidically connected to said fluidic chamber;
- an outlet port fluidically connected to said fluidic chamber;

b) dispensing a flowing liquid growth medium, optionally comprising microorganisms, animal cells, plant cells or fungi cells, into said fluidic chamber, said growth medium flowing across said detachable and/or removable surface, thereby generating a surface-dwelling multicellular layer on said detachable and/or removable surface; and

c) monitoring the surface-dwelling multicellular layer on said detachable and/or removable surface under varying conditions.

In particular embodiments said surface-dwelling multicellular layer composed of microbial cells is a biofilm.

More particularly, the method according to the present invention provides that said varying conditions comprise different types of fluid media, different fluid flow rates, different temperatures, different compounds and/or combinations thereof. In particularly, the monitoring of the multicellular layer may occur upon challenging the multicellular layer with a varying set of conditions. These conditions may be physical conditions such as the temperature or fluid flow rates, and/or chemical conditions where the multicellular layer is submitted to different types of fluid media and/or compounds such as for instance antibiotics, viruses which may be introduced into the flow cell after or at the same time as the growth medium.

Using the device according to the present invention and associated methods for using these, an operator is able to tune several conditions in the device, which may influence the formation and/or cultivation of a surface-dwelling multicellular layer. Several parameters can be varied in the devices according to the present invention including for instance:

a) the contact surface, either the bulk surface material or the coating thereon of the removable surface as already detailed above;

b) the type of fluid medium used to grow the surface-dwelling multicellular layers, which can be any type of liquid characterized by a moderate viscosity such as for instance water, liquid food (e.g. milk), human or animal fluids, laboratory media, wastewater, vegetal extracts, cell extracts, solvents, etc.;

c) the fluid flow typically ranging between 0.1 and 10 ml/min (HRT: ranging between 5 minutes and a few seconds);

d) the temperature for growth of the surface-dwelling multicellular layer, typically ranging between 0°C and 99°C;

e) the type of inoculums used, typically the type of suspension of cells of any concentration injected for a chosen period of time. Also a co-injection of inoculum and medium and/or buffer solution may be envisaged;

f) the sequence of injection of various inoculi and media in different orders;

g) either a unidirectional mode where fresh medium is injected and waste is discarded or a (partial) circular mode where a fixed quantity of medium is circulating during the time of the experiment and is not discarded in a waste bottle may be envisaged.

The cleaning of the device according to the present invention is also an important aspect as cleaning methods such as autoclaving kill all living organisms dwelling in the device. After autoclaving all the parts of the system are removed and washed with mild detergent and distilled water, optionally an ultrasonic treatment followed by extensive cleaning with distilled water can be used to remove any remaining adherent particles in tubes and on fluidic device. This cleaning method is fast and severe and is the safest regarding biosafety issues. Cleaning allows the device to be reused with a new removable surface after cleaning. The device according to the present invention allows such severe cleaning methods. While other, less efficient cleaning methods may be used as well (e.g. using sodium hypochlorite to kill bacteria and sterilize the system, UV or ethanol treatment), these methods are considered less severe compared to autoclaving. Moreover, such less efficient methods may lead to extensive use of potentially harmful oxidizing chlorinated substances which can be avoided with autoclaving.

In yet another embodiment the present invention also relates to a flow distributor for distributing a fluidic flow from a narrow inlet channel to a wider fluidic channel, wherein said flow distributor comprises a first distribution region shaped as an isosceles trapezoid fluidically connected through the larger of the two parallel sides of said isosceles trapezoid to a second rectangular shaped distribution region, wherein the narrow inlet channel is fluidically connected to the smaller of the two parallel sides of said first distribution region and said wider fluidic channel is fluidically connected to the second distribution region, **characterized in that** said second distribution region slopes downward relative to the horizontal position of said wider fluidic channel.

More particularly, the second distribution region slopes downward relative to the horizontal position of the first distribution region. More particularly, the slope angle relative to the direction of the first distribution region ranges between 15° and 75°, more particularly between 30° and 60°, more particularly between 40° and 50° and more particularly, about 45°.

In a particular embodiment the slope angle relative to the direction of the first distribution region of the bottom surface of the second distribution region is larger compared to the slope angle relative to the direction of the first distribution region of the top surface of the second distribution region. More particularly, the slope angle relative to the direction of the first distribution region of the top surface of the second distribution region ranges between 15° and 75°, more particularly between 30° and 60°, more particularly between 40° and 50° and more particularly, about 45°, whereas the slope angle relative to the direction of the first distribution region of the bottom surface of the second distribution region is larger than 50°, 60°, 75°, 80°, 85° and more particularly, about 90°.

In a particular embodiment the slope angle relative to the direction of the first distribution region of the top surface of the second distribution region is 45°, while the slope angle relative to the direction of the first distribution region of the bottom surface of the second distribution region is 90°.

The angles of the top and bottom surfaces of the of the second distribution region are chosen to allow an even and efficiently spread of the liquid, while maintaining a good hydrodynamic profile.

**EXAMPLES**

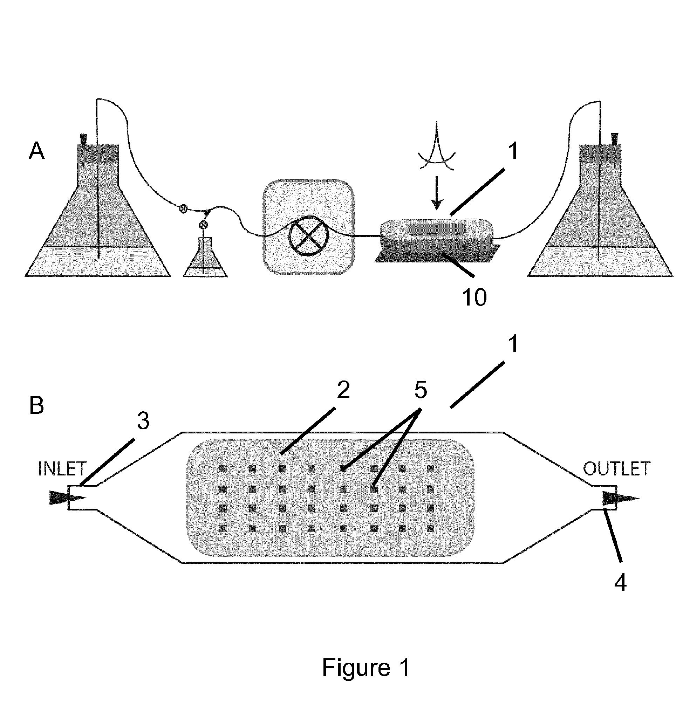

Figure 1 illustrates a specific embodiment of the fluidic device according to the present invention. The fluidic device (1) is incorporated into a fluid flow system (Fig. 1A) where fresh medium is flown through the fluidic device using a pumping system. The fluidic device (1) comprises a fluidic chamber (2), an inlet (3) and an outlet (4). One of the surfaces of the fluidic chamber is removable and on this removable surface (10) formation of the surface-dwelling multicellular layer is monitored through an array of observation zones (5) as shown in Fig. 1B.

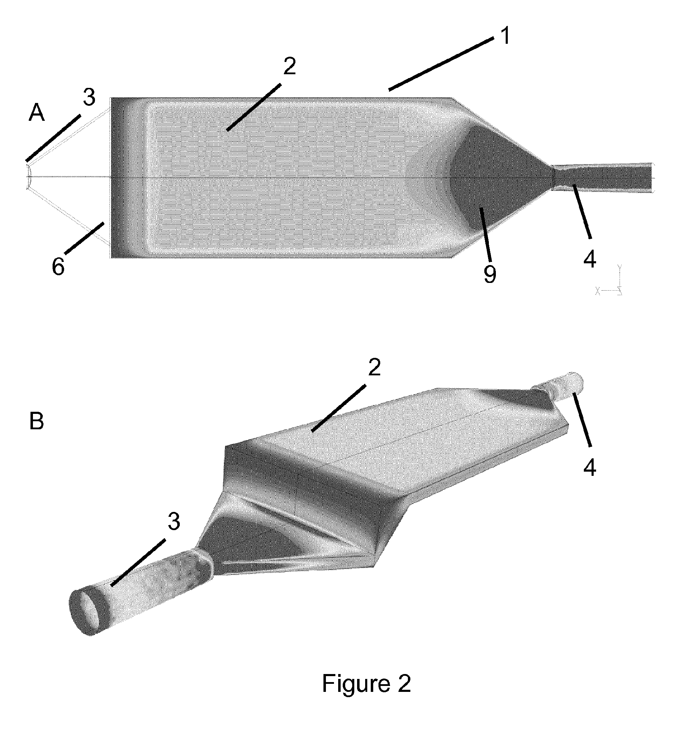

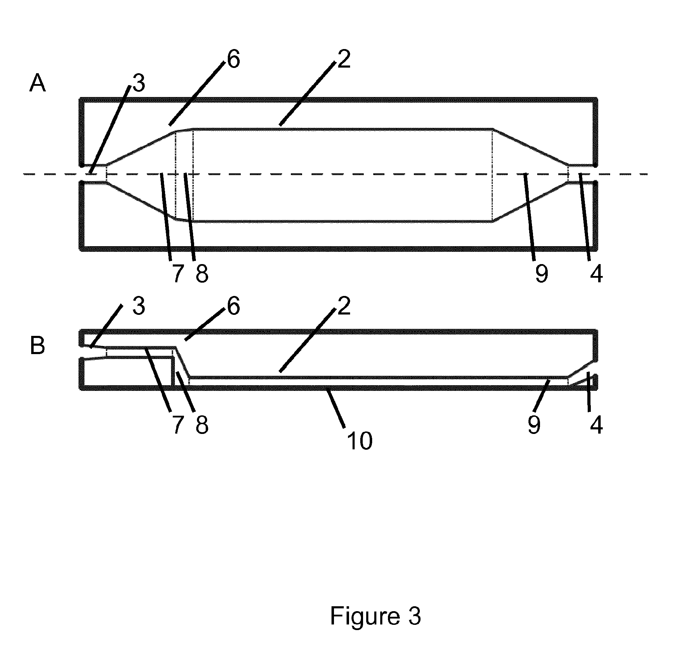

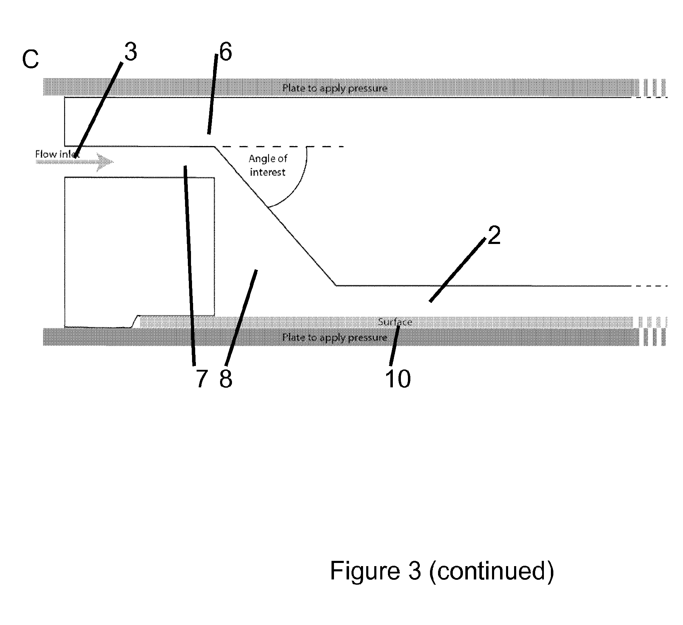

Figure 2 shows fluidic simulations (Fluent) in a fluidic device (1) according to an embodiment of the present invention comprising a fluidic chamber (2), an inlet (3) and an outlet (4). The fluidic device is also provided with an inlet flow distributor (6) and an outlet flow distributor (9). The simulations show that the fluid flow is spread evenly in the fluidic chamber so that there is no dead zone in the chamber. The numerical analysis also shows that both speed profile and shear forces are constant on the entirety of the zone where the analysis of the multicellular layer occurs (a 25 x 15mm zone in the center of the fluidic chamber). This ensures a fine-tuned control over experimental conditions in order to yield reproducible formation of the multicellular layer. In addition, the whole analysis area can be divided into a number of observation spots (as shown in figure 1B) considered to be repetitions from one another which makes the system interesting from the statistical point of view. Figure 3A, 3B and 3C illustrate a specific embodiment of the fluidic device according to the present invention. The fluidic device (1) comprises a fluidic chamber (2), an inlet (3) and an outlet (4). The fluidic device is also provided with an inlet flow distributor (6) and an outlet flow distributor (9). One of the surfaces of the fluidic chamber is removable and on this removable surface (10) formation of the surface-dwelling multicellular layer is monitored. The inlet flow distributor (6) comprises a first distribution region (7) shaped as an isosceles trapezoid fluidically connected through the larger of the two parallel sides of said isosceles trapezoid to a second rectangular shaped distribution region (8), wherein the narrow inlet channel is fluidically connected to the smaller of the two parallel sides of said first distribution region and said wider fluidic channel is fluidically connected to the second distribution region, **characterized in that** said second distribution region slopes downward relative to the horizontal position of said wider fluidic channel. More particularly, the slope angle relative to the direction of the first distribution region of the top surface of the second distribution region is 45°, while the slope angle relative to the direction of the first distribution region of the bottom surface of the second distribution region is 90°.

**Claims**

1. A fluidic device for cultivation and/or analysis of surface-dwelling multicellular layers comprising:

  - a fluidic chamber comprising one or more surfaces enclosing the fluidic chamber through which fluid can flow along a flow path, wherein said fluidic chamber is **characterized by** having a Young's Modulus [E] ranging between 500 kPa and 1 MPa;
  - an inlet port fluidically connected to said fluidic chamber;

- an outlet port fluidically connected to said fluidic chamber;

characterized therein that said fluidic chamber comprises at least one detachable and/or removable surface for the cultivation and/or analysis of surface-dwelling multicellular layers.

2. A fluidic device according to claim 1, wherein the flow of the fluid through said fluidic chamber is laminar.

3. A fluidic device according to claim 1 or 2, wherein the flow of the fluid through said fluidic chamber provides a linear flow profile over the entire width of the fluidic chamber, with the exception of the area 10% from the side walls.

4. A fluidic device according to any of claims 1 to 3, wherein said fluidic chamber comprises a bottom surface, two side surfaces and a top surface, wherein at least part of said top or bottom surface are detachable and/or removable.

5. A fluidic device according to any of claims 1 to 4, wherein at least part of said fluidic chamber is transparent for optical imaging, for microscopy, and/or for fluorescence imaging, thereby providing an imaging observation site.

6. A fluidic device according to any of claims 1 to 5, further comprising observation zones and/or sensors located on or in one or more surface enclosing said fluidic chamber, for observing and/or monitoring the formation and/or cultivation of the surface-dwelling multicellular layer on said detachable and/or removable surface.

7. A fluidic device according to any of claims 1 to 6, wherein said detachable and/or removable surface is made from or provided with an adherent surface material suitable for adhering a surface-dwelling multicellular layer, wherein said surface material models a surface likely to be involved in cell adhesion and/or formation of the multicellular layer.

8. A fluidic device according to any of claims 1 to 7, wherein said adherent surface material is chosen from the group comprising aluminum, stainless steel, silver, copper, hydroaxyapatite, silicon, latex, urethane, PVC, ceramic, steel, gold, titanium, polyethylene, polysiloxanes, biocompatible glasses, polymethylmethacrylate, Teflon (or PTFE), polypropylene, polystyrene, polyamides, polyethers, polyesters, coated block polymers of polyethylene oxide (PEO), polypropylene oxide (PPO), polybutylene oxide (PBO), hydrogels, food film polymers, polycarbonate filters and minerals.

9. A fluidic device according to any of claims 1 to 8, wherein said fluidic device further comprising an inlet flow distributor for distributing incoming fluid over the fluidic chamber and optionally an outlet flow distributor for guiding the outflowing fluid from the fluidic chamber to the outlet.

10. A fluidic device according to claim 9, wherein said inlet flow distributor or part thereof slopes downward relative to the horizontal position of the fluidic chamber.

11. A fluidic device according to any of claims 1 to 10, wherein the depth of the fluidic chamber ranges between 0.1 and 5 mm.

12. A fluidic device according to any of claims 1 to 10, wherein said fluidic device is made from a polymeric material and wherein said detachable and/or removable surface wall of said fluidic device is made from a material other than said polymeric material.

13. A method for cultivating and monitoring surface-dwelling multicellular layers, comprising:

a) providing a fluidic device comprising:

- a fluidic chamber comprising one or more surfaces enclosing the fluidic chamber through which fluid can flow along a flow path, said fluidic chamber comprising at least one detachable and/or removable surface for the cultivating multicellular layers;
- an inlet port fluidically connected to said fluidic chamber;
- an outlet port fluidically connected to said fluidic chamber;

b) dispensing a flowing liquid growth medium, optionally comprising microorganisms, animal cells, plant cells or fungi cells, into said fluidic chamber, said growth medium flowing across said detachable and/or removable surface, thereby generating a surface-dwelling multicellular layer on said detachable and/or removable surface; and

c) monitoring the multicellular layer on said detachable and/or removable surface under varying conditions.

14. Method according to claim 13, wherein said varying conditions comprise different types of fluid media, different fluid flow rates, different temperatures, different compounds and/or combinations thereof.

15. A flow distributor for distributing a fluidic flow from a narrow inlet channel to a wider fluidic channel, wherein said flow distributor comprises a first distribution region shaped as an isosceles trapezoid fluidically connected through the larger of the two parallel sides of said isosceles trapezoid to a second rectangular shaped distribution region, wherein the narrow inlet channel is fluidically connected to the smaller of the two parallel sides of said first distribution region and said wider fluidic channel is fluidically connected to the second distribution region, **characterized in that** said second distribution region slopes downward relative to the horizontal position of said wider fluidic channel.

Figure 1

Figure 2

Figure 3

Figure 3 (continued)

Europäisches
Patentamt

European
Patent Office

Office européen
des brevets

# EUROPEAN SEARCH REPORT

Application Number

EP 12 18 3279

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 468 846 A1 (LUDWIG-MAXIMILIANS-UNIVERSITÄT) 27 June 2012 (2012-06-27) * paragraphs [0001], [0010], [0016], [0018], [0021], [0034], [0036] - [0039], [0041] - [0043], [0045], [0047], [0048], [0050], [0053] * * figures 1-5 * | 1-7,9, 11,13,14 | INV. C12M3/04 |
| X | DE 20 2006 012978 U1 (FORSCHUNGSZENTRUM KARLSRUHE GMBH) 7 December 2006 (2006-12-07) * paragraphs [0001], [0006] - [0008], [0033]; figure 1 * | 1-7,9, 11-14 | |
| X | EP 0 060 509 A2 (PETERSON ET AL.) 22 September 1982 (1982-09-22) * page 1, paragraph 1 * * page 3 - page 4 * * page 5, paragraph 6; figure 1A * | 1,2,4-8, 13,14 | |
| X | DE 199 33 018 A1 (UNIVERSITÄTSKLINIKUM FREIBURG) 18 January 2001 (2001-01-18) * page 3, line 31 - line 56; claims 1-4 * | 1-4,9, 11,13,14 | TECHNICAL FIELDS SEARCHED (IPC) C12M |
| X | US 2011/212493 A1 (HIRSCHEL ET AL.) 1 September 2011 (2011-09-01) * figures 1A-2B * * paragraphs [0013], [0020], [0021], [0025], [0032], [0034] - [0036] * | 1-4,7,9, 11,13,14 | |
| A | EP 0 747 475 A2 (BECTON DICKINSON AND COMPANY) 11 December 1996 (1996-12-11) * column 1 - column 2 * * column 4, line 30 - line 50; figures 1-5 * | 1,12 | |

-/--

~~The present search report has been drawn up for all claims~~

| Place of search | Date of completion of the search | Examiner |
|---|---|---|
| Berlin | 9 January 2013 | Alvarez Alvarez, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**

**European Patent Office**

**Office européen des brevets**

# EUROPEAN SEARCH REPORT

Application Number

EP 12 18 3279

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 5 565 353 A (KLEBE ET AL.) 15 October 1996 (1996-10-15) * column 1, line 8 - line 14; figures 1-4 * ----- | 1,12 | |
| | | | TECHNICAL FIELDS SEARCHED (IPC) |
| | The present search report has been drawn up for all claims | | |

| Place of search | Date of completion of the search | Examiner |
|---|---|---|
| Berlin | 9 January 2013 | Alvarez Alvarez, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                           
& : member of the same patent family, corresponding document

EPO FORM 1503 03.82 (P04C01)

Europäisches
Patentamt

European
Patent Office

Office européen
des brevets

**Application Number**

EP 12 18 3279

---

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-14

☐ The present supplementary European search report has been drawn up for those parts of the European patent application which relate to the invention first mentioned in the claims (Rule 164 (1) EPC).

Europäisches
Patentamt
European
Patent Office
Office européen
des brevets

**LACK OF UNITY OF INVENTION
SHEET B**

Application Number

EP 12 18 3279

The Search Division considers that the present European patent application does not comply with the requirements of unity of invention and relates to several inventions or groups of inventions, namely:

1. claims: 1-14

   A fluidic device for cultivation and/or analysis of surface-dwelling multicellular layers comprising:
   - a fluidic chamber comprising one or more surfaces enclosing the fluidic chamber through which fluid can flow along a flow path, wherein said fluidic chamber is characterized by having a Young's Modulus [E] ranging between 500 kPa and 1 MPa;
   - an inlet port fluidically connected to said fluidic chamber;
   - an outlet port fluidically connected to said fluidic chamber;
   characterized therein that said fluidic chamber comprises at least one detachable and/or removable surface for the cultivation and/or analysis of surface-dwelling multicellular layers.
   A method for cultivating and monitoring surface-dwelling multicellular layers, comprising:
   a) providing a fluidic device comprising:
   - a fluidic chamber comprising one or more surfaces enclosing the fluidic chamber through which fluid can flow along a flow path, said fluidic chamber comprising at least one detachable and/or removable surface for the cultivating multicellular layers;
   - an inlet port fluidically connected to said fluidic chamber;
   - an outlet port fluidically connected to said fluidic chamber;
   b) dispensing a flowing liquid growth medium, optionally comprising microorganisms, animal cells, plant cells or fungi cells, into said fluidic chamber, said growth medium flowing across said detachable and/or removable surface, thereby generating a surface-dwelling multicellular layer on said detachable and/or removable surface; and
   c) monitoring the multicellular layer on said detachable and/or removable surface under varying conditions.
   ---

2. claim: 15

   A flow distributor for distributing a fluidic flow from a narrow inlet channel to a wider fluidic channel, wherein said flow distributor comprises a first distribution region shaped as an isosceles trapezoid fluidically connected through the larger of the two parallel sides of said isosceles trapezoid to a second rectangular shaped distribution region, wherein the narrow inlet channel is fluidically connected to the smaller of the two parallel sides of said first distribution region and said wider fluidic channel is fluidically connected to the second

Europäisches
Patentamt

European
Patent Office

Office européen
des brevets

**LACK OF UNITY OF INVENTION**
**SHEET B**

Application Number

EP 12 18 3279

The Search Division considers that the present European patent application does not comply with the requirements of unity of invention and relates to several inventions or groups of inventions, namely:

distribution region, characterized in that said second distribution region slopes downward relative to the horizontal position of said wider fluidic channel.
---

**ANNEX TO THE EUROPEAN SEARCH REPORT**

**ON EUROPEAN PATENT APPLICATION NO.** EP 12 18 3279

This annex lists the patent family members relating to the patent documents cited in the above-mentioned European search report.
The members are as contained in the European Patent Office EDP file on
The European Patent Office is in no way liable for these particulars which are merely given for the purpose of information.

09-01-2013

| Patent document cited in search report | | Publication date | Patent family member(s) | | Publication date |
|---|---|---|---|---|---|
| EP 2468846 | A1 | 27-06-2012 | EP 2468846 A1 | | 27-06-2012 |
| | | | WO 2012084212 A1 | | 28-06-2012 |
| DE 202006012978 | U1 | 07-12-2006 | NONE | | |
| EP 60509 | A2 | 22-09-1982 | DE 3267490 D1 | | 02-01-1986 |
| | | | EP 0060509 A2 | | 22-09-1982 |
| | | | EP 0073773 A1 | | 16-03-1983 |
| | | | JP S58500234 A | | 17-02-1983 |
| | | | SE 448881 B | | 23-03-1987 |
| | | | SE 8101564 A | | 13-09-1982 |
| | | | US 4530907 A | | 23-07-1985 |
| | | | WO 8203227 A1 | | 30-09-1982 |
| DE 19933018 | A1 | 18-01-2001 | NONE | | |
| US 2011212493 | A1 | 01-09-2011 | AU 2009308354 A1 | | 29-04-2010 |
| | | | CA 2741481 A1 | | 29-04-2010 |
| | | | EP 2346984 A2 | | 27-07-2011 |
| | | | JP 2012506257 A | | 15-03-2012 |
| | | | US 2011212493 A1 | | 01-09-2011 |
| | | | WO 2010048417 A2 | | 29-04-2010 |
| EP 747475 | A2 | 11-12-1996 | CA 2172343 A1 | | 07-12-1996 |
| | | | DE 69618384 D1 | | 14-02-2002 |
| | | | DE 69618384 T2 | | 26-09-2002 |
| | | | EP 0747475 A2 | | 11-12-1996 |
| | | | JP 2891334 B2 | | 17-05-1999 |
| | | | JP H08332073 A | | 17-12-1996 |
| | | | US 5618731 A | | 08-04-1997 |
| US 5565353 | A | 15-10-1996 | NONE | | |

For more details about this annex : see Official Journal of the European Patent Office, No. 12/82